# EUROPEAN PATENT SPECIFICATION

(11) **EP 4 427 670 B1**
(45) Date of publication and mention of the grant of the patent: **02.12.2026**
(21) Application number: 22890212.8
(22) Date of filing: 14.10.2022
(51) Int. Cl.: A61B 5/055, A61B 5/026, A61B 5/00, G01R 33/563, G01R 33/56, G01R 33/48, G16H 50/20, G06T 7/11, G16H 30/40

(54) **METHOD AND ANALYTIC DEVICE FOR COLLATERAL CIRCULATION ASSESSMENT, USING DEEP LEARNING MODEL-BASED DYNAMIC PERFUSION MRI IMAGE**
VERFAHREN UND ANALYSEVORRICHTUNG ZUR KOLLATERALEN KREISLAUFBEURTEILUNG UNTER VERWENDUNG EINES MODELLBASIERTEN DYNAMISCHEN PERFUSIONS-MRT-BILDES FÜR TIEFES LERNEN
PROCÉDÉ ET DISPOSITIF ANALYTIQUE POUR ÉVALUATION DE CIRCULATION COLLATÉRALE, UTILISANT UNE IMAGE IRM DE PERFUSION DYNAMIQUE BASÉE SUR UN MODÈLE D'APPRENTISSAGE PROFOND

(30) Priority: 03.11.2021 KR 20210149615
(43) Date of publication of application: 11.09.2024
(73) Proprietor: Samsung Life Public Welfare Foundation, Seoul 04348 (KR)
(72) Inventor: BANG, Oh Young, Seoul 06351 (KR); KIM, Yoon Chul, Seoul 06351 (KR); CHUNG, Jong Won, Seoul 06351 (KR); SEO, Woo Keun, Seoul 06351 (KR); KIM, Gyeong Moon, Seoul 06351 (KR)
(74) Representative: Ostertag & Partner Patentanwälte mbB
(86) International application number: PCT/KR2022/015553
(87) International publication number: WO 2023/080477

(56) References cited:
- WO-A1-2010/058014
- KR-A- 20140 142 652
- KR-A- 20200 113 336
- US-A1- 2007 167 731
- US-A1- 2016 125 597
- XIAO YIMING ET AL: "Towards Automatic Collateral Circulation Score Evaluation in Ischemic Stroke Using Image Decompositions and Support Vector Machines", 9 September 2017, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 158 - 167, ISBN: 978-3-540-74549-5, XP047439834
- AKTAR MUMU ET AL: "A Radiomics-Based Machine Learning Approach to Assess Collateral Circulation in Ischemic Stroke on Non-contrast Computed Tomography", 1 October 2020, 20201001, PAGE(S) 24 - 33, XP047564481
- ANONYMOUS: "A Gentle Introduction to Ensemble Learning Algorithms", 19 April 2021 (2021-04-19), pages 1 - 12, XP093297998, Retrieved from the Internet <URL:https://web.archive.org/web/20210421161038/https://machinelearningmastery.com/tour-of-ensemble-learning-algorithms/> [retrieved on 20250721]
- ROJARATH ARTITTAYAPRON ET AL: "Improved ensemble learning for classification techniques based on majority voting", 2016 7TH IEEE INTERNATIONAL CONFERENCE ON SOFTWARE ENGINEERING AND SERVICE SCIENCE (ICSESS), IEEE, 26 August 2016 (2016-08-26), pages 107 - 110, XP033079835, DOI: 10.1109/ICSESS.2016.7883026
- TO MINH NGUYEN NHAT ET AL: "Deep regression neural networks for collateral imaging from dynamic susceptibility contrast-enhanced magnetic resonance perfusion in acute ischemic stroke", INTERNATIONAL JOURNAL OF COMPUTER ASSISTED RADIOLOGY AND SURGERY, SPRINGER, DE, vol. 15, no. 1, 3 September 2019 (2019-09-03), pages 151 - 162, XP036982832, ISSN: 1861-6410, [retrieved on 20190903], DOI: 10.1007/S11548-019-02060-7
- GILES TETTEH; FERNANDO NAVARRO; JOHANNES PAETZOLD; JAN KIRSCHKE; CLAUS ZIMMER; BJOERN H. MENZE: "A Deep Learning Approach to Predicting Collateral Flow in Stroke Patients Using Radiomic Features from Perfusion Images", ARXIV.ORG, CORNELL UNIVERSITY LIBRARY, 201 OLIN LIBRARY CORNELL UNIVERSITY ITHACA, NY 14853, 24 October 2021 (2021-10-24), XP091080853

## Description

### Technical Field

The following description relates to a collateral circulation assessment technique using perfusion magnetic resonance imaging (MRI).

### Background Art

In patients with acute ischemic stroke, both antegrade flow and retrograde collateral flow maintain cerebral blood flow within an ischemic lesion. Brain magnetic resonance imaging (MRI) is widely used to assess collateral circulation. In addition to general MRI, perfusion MRI may be used to assess collateral circulation. US 2016/125597 A1 describes a technique for processing dynamic susceptibility contrast perfusion magnetic resonance imaging (DSC-PWI) data in order to generate images associated with collateral circulation.
Xiao Yiming, Zhang X., et al., "Towards Automatic Collateral Circulation Score Evaluation in Ischemic Stroke Using Image Decompositions and Support Vector Machines", 9 September 2017 (2017-09-09), SAT 2015 18th International Conference, Austin, Tx, Usa, September 24-27, 2015; [Lecture Notes In Computer Science; Lect.Notes Computer], Springer, Berlin, Heidelberg, Page(S) 158-167 describe an automated collateral circulation scoring approach based on four-dimensional computed tomography angiography (4D-CTA) data. The method includes image decomposition and projection steps, extraction of quantitative features, and classification using support vector machines (SVM)

### Disclosure

### Technical Problem

The scope of protection is limited to the appended claims. The following description provides a technique for automatically assessing collateral circulation using perfusion magnetic resonance imaging (MRI).

### Technical Solution

In one general aspect, there is provided a method of collateral circulation assessment of a subject using dynamic perfusion magnetic resonance imaging (MRI) images includes receiving, by an analysis device, dynamic perfusion MRI images of a brain of a subject, extracting, by the analysis device, arterial phase images, capillary phase images, and venous phase images from the dynamic perfusion MRI images, inputting, by the analysis device, each of the arterial phase images, the capillary phase images, and the venous phase images into a pre-trained deep learning model to calculate a plurality of collateral circulation prediction probability values, and performing, by the analysis device, a final collateral circulation assessment for the subject using the plurality of collateral circulation prediction probability values. The references to the methods of treatment by therapy or surgery or in vivo diagnosis methods are disclosed here only for the purpose of explaining the invention but do not fall within the scope of the claims.

In another aspect, there is provided an analysis device for collateral circulation assessment of a subject using dynamic perfusion MRI images includes an input device that receives dynamic perfusion MRI images of a brain of a subject, a storage device that stores a deep learning model trained to calculate a collateral circulation state probability value for an input MRI slice, and a calculation device that extracts arterial phase images, capillary phase images, and venous phase images from the dynamic perfusion MRI images, inputs each of the arterial phase images, the capillary phase images, and the venous phase images to the deep learning model to calculate a plurality of collateral circulation prediction probability values, and performs a final collateral circulation assessment for the subject using the plurality of collateral circulation prediction probability values.

### Advantageous Effects

The following description can automatically assess collateral circulation using a patient's perfusion MRI using a deep learning model. As a result, the following description is possible to help provide guidance for patient care in emergency situations without the help of a reading expert.

### Description of Drawings

FIG. 1 is an example of a system for collateral circulation assessment using brain magnetic resonance imaging (MRI).
FIG. 2 is an example of a process of collateral circulation assessment using brain MRI.
FIG. 3 is an example of a process of generating a collateral image composed of an arterial phase, a capillary phase, and a venous phase using dynamic perfusion MRI.
FIG. 4 is an example of an R₂* time intensity curve (TIC).
FIG. 5 is an example of a process of performing collateral circulation assessment using a deep learning model.
FIG. 6 is another example of a process of performing collateral circulation assessment using a deep learning model.
FIG. 7 is an example of an analysis device for performing collateral circulation assessment using dynamic perfusion MRI.

### Exemplary embodiments of invention

A technology to be described below may be variously modified and have several exemplary embodiments. Therefore, specific exemplary embodiments of the present disclosure will be illustrated in the accompanying drawings and be described in detail.

Terms such as "first," "second," "A," "B," and the like may be used to describe various components, but the components are not to be interpreted as limited to the terms, which are used only for distinguishing one component from other components. For example, a first component may be named a second component and a second component may also be similarly named a first component without departing from the scope of the technologies described below. The term "and/or" includes a combination of a plurality of related described items or any one of the plurality of related described items.

It should be understood that singular expressions include plural referents unless the context clearly indicates otherwise, and it will be further understood that terms such as "comprise" and "have" used in this specification specify the presence of stated features, steps, operations, components, parts, or a combination thereof, but do not preclude the presence or addition of one or more other features, numerals, steps, operations, components, parts, or a combination thereof.

Prior to the detailed description of the drawings, it is intended to clarify that the components in this specification are only distinguished by their main functions. That is, two or more components to be described below may be combined into one component, or one component may be divided into two or more for each subdivided function. In addition, each of the constituent parts to be described below may additionally perform some or all of the functions of other constituent parts in addition to the main functions of the constituent parts, and some of the main functions of the constituent parts may be performed exclusively by other components.

In addition, in performing the method or the operation method, each of the processes constituting the method may occur differently from the specified order unless a specific order is explicitly described in context. That is, the respective steps may be performed in the same sequence as the described sequence, performed at substantially the same time, or performed in an opposite sequence to the described sequence.

The technique described below is a technique to assess collateral circulation in a brain. The collateral circulation is a phenomenon in which blood flow bypasses a blockage in normal blood flow due to vascular stenosis or occlusion through other blood vessels. Cerebral blood flow is composed of anterior circulation by the bilateral carotid arteries and posterior circulation by the vertebral basilar artery on the back, and the anterior circulation and the posterior circulation anastomose at a base part of a brain to form the circle of Willis, and branch into the anterior cerebral artery, the middle cerebral artery, and the posterior cerebral artery. Each of these blood vessels is ultimately responsible for supplying blood to a certain area of the brain, but anastomoses between these blood vessels are also formed in several places to achieve collateral circulation in the event of a lesion of one blood vessel.

The technology described below uses perfusion magnetic resonance imaging (MRI) images. The perfusion phenomenon involves delivering blood to parenchyma of cells through capillaries. In the perfusion MRI, an exogenous perfusion MRI method obtained by administering a contrast agent through a vein in a human body has been commonly used. In other words, it is a method of continuously obtaining images from a certain body part while tracking a flow of contrast agent over time.

The dynamic perfusion MRI is a technique of continuously obtaining multiple frames of brain images for 1 to 2 minutes with a temporal resolution of 1 to 2 seconds during a first pass of a contrast agent. In the brain images, perfusion-weighted images, which are blood flow images using a signal reduction method, mainly use a dynamic susceptibility contrast (DSC) technique. A DSC image is obtained as a T2*-weighted image since the contrast agent changes T2* by making a difference in surrounding susceptibility large. In the case of brain tissue suffering from ischemia due to blocked cerebral blood vessels, the contrast agent is not supplied well to the tissues, and therefore the MRI image is brighter than that of normal brain tissue during the first pass, and the point at which the MRI signal is minimized is delayed compared to normal brain tissues.

An arterial phase is a phase in which contrast dye staining begins from an insular region of a normal brain parenchyma, a capillary phase is a phase in which the entire brain parenchyma on the normal side is stained with the highest signal intensity, and a venous phase is a phase in which the contrast agent is washed out from the normal brain parenchyma.

Hereinafter, it is described that the analysis device assesses collateral circulation in the dynamic perfusion MRI images using a certain learning model. The analysis device may be implemented as a variety of devices capable of processing data. For example, the analysis device may be implemented as a personal computer (PC), a server on a network, a smart device, or a chipset with a dedicated program embedded therein, etc.

Examples of the learning model include a decision tree, a random forest (RF), a K-nearest neighbor (KNN), a naive Bayes, a support vector machine (SVM), an artificial neural network (ANN), and the like. In particular, the technology described below may assess the collateral circulation in the dynamic perfusion MRI images using an ANN.

An ANN is a statistical learning algorithm that mimics a biological neural network. Various neural network models are being studied. Recently, a deep learning network (DNN) has been attracting attention. A DNN is an ANN model composed of several hidden layers between an input layer and an output layer. Similar to general ANNs, a DNN may model complex non-linear relationships. Various types of DNN models have been studied. Examples of DNNs include a convolutional neural network (CNN), a recurrent neural network (RNN), a restricted Boltzmann machine (RBM), a deep belief network (DBN), a generative adversarial network (GAN), reinforcement learning (RL), and the like.

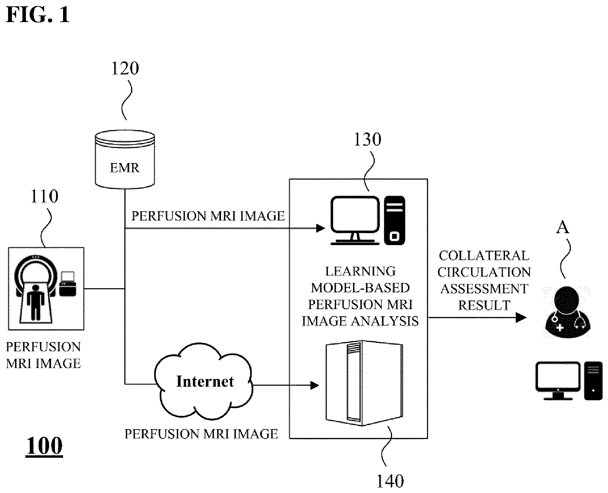

FIG. 1 is an example of a system 100 for collateral circulation assessment using brain MRI. In FIG. 1, the analysis device illustrates an example of a computer terminal 130 and a server 140.

The MRI equipment 110 generates brain MRI images for a subject. In particular, the MRI equipment 110 may generate perfusion MRI images or dynamic perfusion MRI images for a subject into which the contrast agent is injected. The perfusion MRI images of the subject may be stored in an electronic medical record (EMR) 120.

In FIG. 1, a user A may use the computer terminal 130 to assess the collateral circulation using the perfusion MRI images of the subject. The computer terminal 130 receives the perfusion MRI images of the subject. The computer terminal 130 may receive the perfusion MRI images from the MRI equipment 110 or the EMR 120 through a wired or wireless network. In some cases, the computer terminal 130 may be a device physically connected to the MRI equipment 110.

The computer terminal 130 may consistently preprocess the perfusion MRI images of the subject. The computer terminal 130 may generate arterial phase images, capillary phase images, and venous phase images based on the perfusion MRI images. The computer terminal 130 may input the arterial phase images, the capillary phase images, and the venous phase images to a pre-trained deep learning model to derive the collateral circulation assessment results for the subject. The detailed analysis process will be described below. The user A may check the analysis results.

The server 140 may receive the perfusion MRI images of the subject from the MRI equipment 110 or the EMR 120. The server 140 may consistently process the perfusion MRI images of the subject. The server 140 may generate the arterial phase images, the capillary phase images, and the venous phase images based on the perfusion MRI images. The server 140 may input the arterial phase images, the capillary phase images, and the venous phase images to the pre-trained deep learning model to derive the collateral circulation assessment results for the subject. The detailed analysis process will be described below. The server 140 may transmit the analysis results to the user A's terminal. The user A may check the analysis results.

The computer terminal 130 and/or server 140 may store the analysis results in the EMR 120.

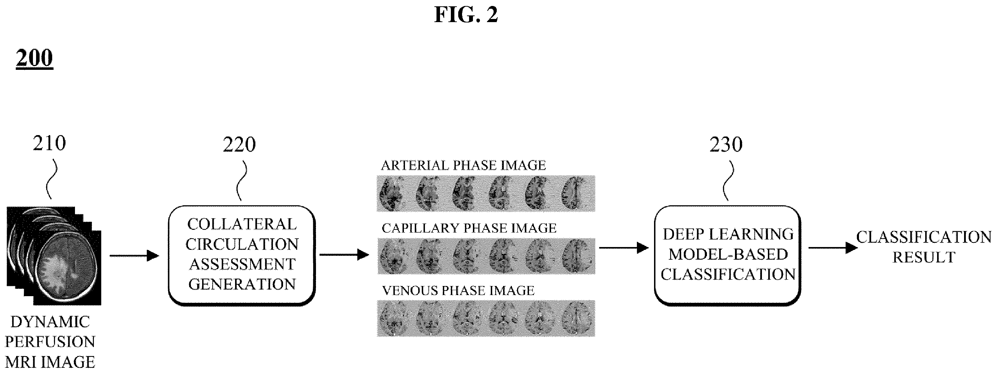

FIG. 2 is an example of a collateral circulation assessment process 200 using dynamic perfusion MRI.

The analysis device receives the dynamic perfusion MRI images of the brain of the subject (210). Detailed description of the process of acquiring the dynamic perfusion MRI images will be omitted.

The analysis device generates the arterial phase images, the capillary phase images, and the venous phase images using the dynamic perfusion MRI images of the subject (220). The analysis device consistently processes raw data of the dynamic perfusion MRI images to generate the arterial phase images, the capillary phase images, and the venous phase images. The detailed process will be described below.

The analysis device inputs the generated arterial phase images, capillary phase images, and venous phase images to the pre-trained deep learning model to assess the collateral circulation (230). The deep learning model extracts features from the input images and calculates a predicted value (probability value) for the collateral circulation state based on the extracted features.

Meanwhile, the deep learning model may be implemented in various structures. For example, the deep learning model may receive the arterial phase images, the capillary phase images, and the venous phase images through individual input layers, extract features from each image at a separate convolution layer, connect the individual features, and calculate the probability value for the collateral circulation through a fully connected layer. Alternatively, the deep learning model may build individual models for each of the arterial phase images, capillary phase images, and venous phase images, and use the values calculated by each individual model to assess a final collateral circulation state.

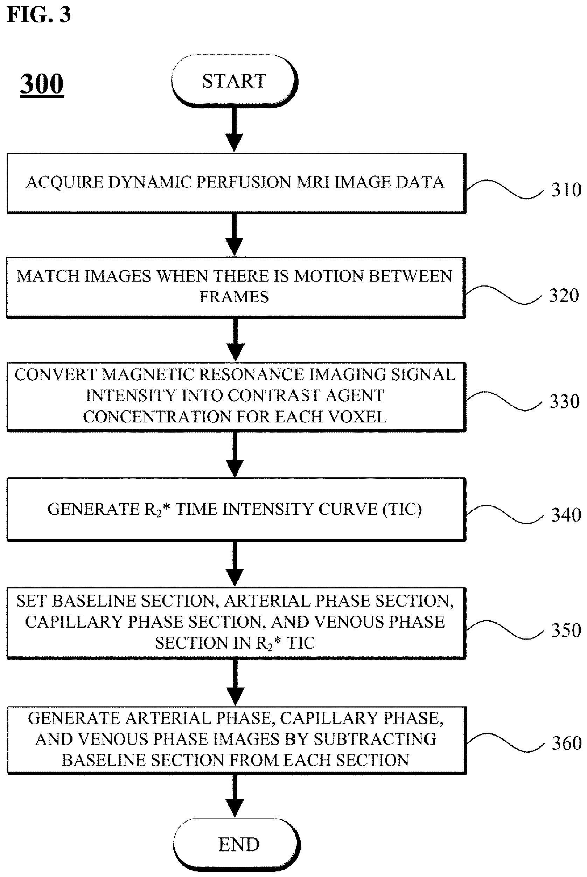

FIG. 3 is an example of a process 300 of generating a collateral image composed of an arterial phase, a capillary phase, and a venous phase using dynamic perfusion MRI.

The analysis device acquires data of the dynamic perfusion MRI images 310. The dynamic perfusion MRI images may be in the form of a DICOM file. The dynamic perfusion MRI images may be expressed as a four-dimensional array S(x,y,z,t). Here x, y and z are positions in a three-dimensional space, and t refers to time.

When movement of an object occurs between frames, the analysis device may perform image registration to match the positions of the images between the frames (320).

The analysis device converts the MRI signal intensity into the contrast agent concentration for each voxel (330).

The analysis device may perform the following processing in one voxel unit. The analysis device may convert the MRI signal intensity S(t) into contrast agent concentration C(t) for voxels (x, y, z) as shown in Equation 1 below. [Equation 1] $C \left(t\right) = - \frac{1}{{r}_{2} ⋅ TE} ⋅ log \left(\frac{S \left(t\right)}{{S}_{0}}\right)$

Here, r₂ denotes a T2 relaxation rate constant, TE denotes an echo time, and S₀ denotes an initial value of the MRI signal intensity before the contrast agent injection. The relaxation rate R₂*(t) is proportional to the contrast agent concentration C(t) and is calculated by putting R₂*(t) = kC(t) for convenience.

The analysis device generates an R₂* time intensity curve (TIC) (340). The analysis device may obtain the TIC by calculating the TIC for each time frame t with a value obtained by averaging the contrast agent concentrations for all slices or all voxels of a slice in a region of interest, as shown in Equation 2 below. [Equation 2] ${R}_{2}^{∗} \left(t\right) = 1 / N ⋅ {\sum }_{x , y , z} {R}_{2}^{∗} \left(x, y, z, t\right)$

The analysis device sets a baseline section, an arterial phase section, a capillary phase section, and a venous phase section in the generated R₂* TIC (350).

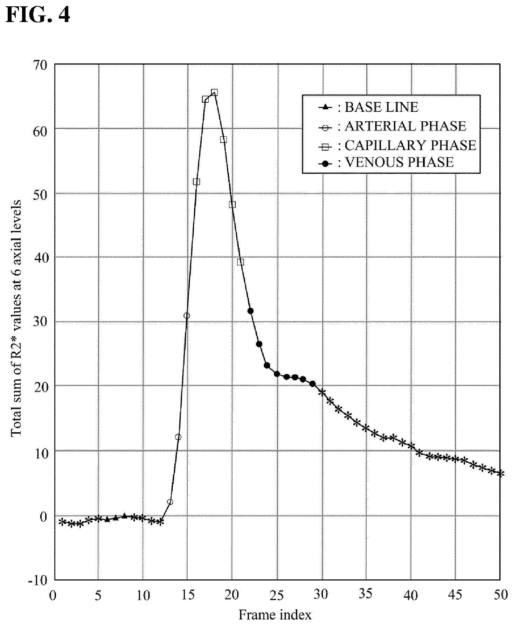

This process will be described based on FIG. 4. FIG. 4 is an example of the R₂* time intensity curve (TIC). In FIG. 4, a horizontal axis denotes a frame index, and a vertical axis denotes an R₂* sum value in the corresponding frame. The analysis device first determines the frame and baseline section with the highest R₂* value in the TIC. The analysis device may set a section (lower half section, section within half of the maximum value) from the point where the curve begins after passing the baseline section to half of the maximum value of R₂* as the arterial phase section. The analysis device may set half (upper half section, section exceeding half of the maximum value) of the maximum value of R₂* as the capillary phase section in the TIC. The analysis device may set the section after the capillary phase section as the venous phase section.

The analysis device subtracts the baseline image from each of the images of the arterial phase section, the capillary phase section, and the venous phase section to generate the final arterial phase images, capillary phase images, and venous phase images 360. (i) The analysis device calculates the baseline image (frame) by subtracting the baseline image from each of the images (frames) located in the arterial phase section. The images calculated in this process are the arterial phase images. (ii) The analysis device calculates the baseline image (frame) by subtracting the baseline image from each of the images (frames) located in the capillary phase section. The images calculated in this process are the capillary phase images. (iii) The analysis device calculates the baseline image (frame) by subtracting the baseline image from each of the images (frames) located in the venous phase section. The images calculated in this process are the venous phase images. Meanwhile, researchers constituted images for each section with adjacent frames for each section.

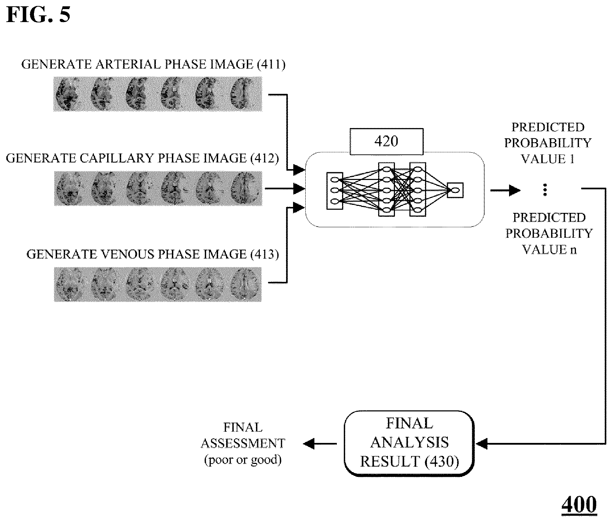

FIG. 5 is an example of a process 400 of performing collateral circulation assessment using a deep learning model. FIG. 5 is an example of a process of performing collateral circulation assessment using one deep learning model.

The learning process will be described briefly. The training data is as described in Table 1 below. Based on the MRI collateral circulation grade assessed by cerebral infarction clinician experts in four grades for each patient, researchers classified grades 1 and 2 as poor, and grades 3 and 4 as good (label data construction). Researchers prepared training image data by augmenting the training data by constantly rotating the training data.

The analysis device generates the arterial phase images (411), the capillary phase images (412), and the venous phase images (413) through the above-described process. The images of each section are composed of a plurality of slices.

The analysis device sequentially inputs each slice of each section to the trained deep learning model (420). When one slice is input, the deep learning model calculates the probability value of the collateral circulation state for the corresponding slice. For example, when there are n slices, n predicted probability values are calculated. Furthermore, when a k-order verification model is used as designed by researchers, a larger number of predicted probability values (n*k) may be calculated.

The analysis device may determine the final probability value by averaging the generated predicted probability values (430). The analysis device may compare the final probability value and a cutoff value to determine the final analysis result (poor or good).

Hereinafter, the results of an experiment for verifying the performance of the deep learning model performing the collateral circulation assessment described above will be described. Table 1 below provides information on data used in the process (model development) of constructing, by researchers, the deep learning model and data used in the verification process (external validation).

**[Table 1]**

| | **Model development (*n*=255)** | | | **External validation (*n*=72)** | | |
|---|---|---|---|---|---|---|
| | **MRP collateral grade** | | ***p*-value** | **MRP collateral grade** | | ***p*-value** |
| | **Poor (*n*=70)** | **Good (*n*=185)** | | **Poor (*n*=28)** | **Good (*n*=44)** | |
| **Age (year)** | 69 (59-75) | 69 (55-77) | 0.99 | 69 (61-80) | 68(61-78) | 0.45 |
| **Men** | 34 (49%) | 104 (56%) | 0.28 | 16 (57%) | 24(55%) | 0.83 |
| **NIHSS baseline** | 18 (16-20) | 14 (10-18) | <0.01 | 17 (13-18) | 13 (9-17) | 0.02 |
| **Onset to MRI time (min)** | 131 (90-232) | 144 (97-231) | 0.71 | 106 (88-145) | 131 (91-236) | 0.48 |
| **Risk factors** | | | | | | |
| **Atrial fibrillation** | 33 (47%) | 67 (36%) | 0.12 | 11 (39%) | 21 (48%) | 0.49 |
| **Hypertension** | 43 (61%) | 107 (58%) | 0.60 | 21 (75%) | 30 (68%) | 0.54 |
| **Diabetes mellitus** | 20 (29%) | 51 (28%) | 0.87 | 9 (32%) | 9(20%) | 0.29 |
| **Dyslipidemia** | 13 (19%) | 61 (33%) | 0.01 | 15 (54%) | 21 (48%) | 0.64 |
| **Current smoking** | 11 (16%) | 31 (18%) | 0.61 | 5 (18%) | 10 (23%) | 0.62 |
| **Stroke mechanism** | | | | | | |
| **Large artery disease** | 10 (14%) | 58 (31%) | <0.01 | 5 (18%) | 10 (22%) | 0.62 |
| **Cardioembolic stroke** | 42 (60%) | 94 (51%) | 0.19 | 18 (64%) | 29 (66%) | 0.89 |
| **Other determined stroke** | 10 (14%) | 22 (12%) | 0.62 | 0 (0%) | 4 (9%) | - |
| **Undetermined stroke** | 8 (11%) | 11 (6%) | 0.20 | 5 (18%) | 1 (2%) | 0.05 |
| **Mode of treatment** | | | | | | |
| **No reperfusion therapy** | 17 (24%) | 35 (19%) | 0.19 | 2 (7%) | 5 (11%) | 0.54 |
| **IV tPA only** | 6 (9%) | 9 (5%) | 0.32 | 2 (7%) | 4 (9%) | 0.77 |
| **EVT±IV tPA** | 47 (67%) | 141 (76%) | 0.16 | 24 (86%) | 35 (80%) | 0.50 |
| **Cath angio collateral grade distribution (0-1/2/3/4)*** | 33/12/6/0 | 28/23/71/30 | <0.001 | 8/12/1/0 | 1/6115/3 | <0.001 |

The researchers used data about 327 patients. The researchers used data about 255 patients as training data to construct the model, and used data about 72 patients as validation data. Table 1 lists clinical information for patients belonging to the poor and good groups. The external validation data includes diffusion-weighted imaging (DWI) and dynamic susceptibility contrast magnetic resonance perfusion (DSC-MRP) scan data before and 7 days after treatment in patients who had undergone successful reperfusion (treatment for cerebral infarction).

The researchers used a CNN model implemented in Keras. The CNN model used was composed of four convolutional layers (each layer including a Relu activation function and a max pooling layer) and two fully connected layers. The deep learning model output binary classification results for poor or good.

The researchers trained the model in the form of fivefold cross-validation. The researchers determined the predicted probability value for the input image as the average of the values calculated by the five models. The researchers derived the cutoff value for the collateral circulation assessment from receiver operating characteristic (ROC) curve analysis.

As a result of external verification, in the deep learning model, an area under the curve (AUC) for good collateral circulation reached 0.85. By using the optimal cutoff value, the statistical analysis results of the consistency between the reader's manual reading results and the results predicted by the deep learning model resulted in a kappa value of 0.53 (95% CI, 0.32-0.73, p<0.0001). Therefore, the performance of the deep learning model performing the collateral circulation assessment was shown to be quite high.

Table 2 below shows the results of assessing the performance of the deep learning model (DL) using the above verification data. First, Table 2 shows the postoperative course and the results predicted by the deep learning model for patients who had undergone the successful reperfusion treatment. Modified treatment in cerebral infarction (mTICI) scores the degree of reperfused area, and a score of 2b or higher means that more than 50% of the existing vascular occlusion area was successfully reperfused. Looking at Table 2, it can be seen that the average infarct growth of patients classified as poor by the deep learning model 7 days after undergoing reperfusion treatment was much larger than that of patients classified as good.

**[Table 2]**

| Group | MR collateral grade | mRS 0-2 at 90 days n (%) | Infarct growth volume (mL) median [IQR] |
|---|---|---|---|
| mTICI 2b-3 | Good | 16 (69) | 6 [1-12.5] |
| | Poor | 15 (60) | 26 [8-74] |
| | p-value | 0.50 | 0.001 |
| mTICI 0-2a or no EVT | Good | 7 (39) | 50 [9-74.5] |
| | Poor | 1 (17) | 59 [6-141] |
| | p-value | 0.30 | 0.50 |

Meanwhile, among the patients included in the validation data, patients with a modified Rankin Scale (mRS) of 0 to 2a for 90 days or patients who did not undergo endovascular therapy (EVT) did not show significant differences in average infarct extension regardless of the results classified by the deep learning model.

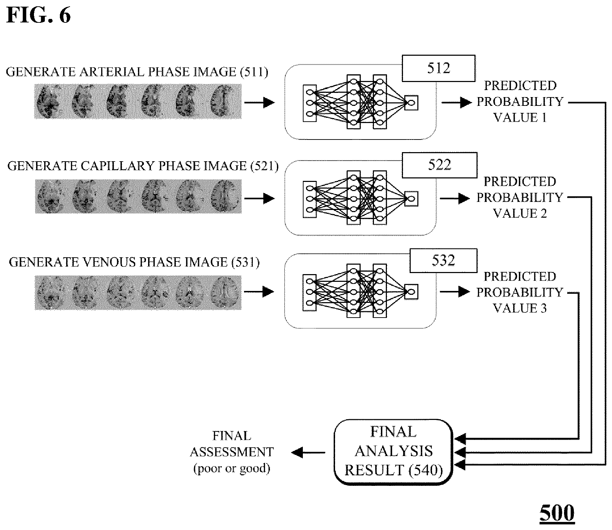

FIG. 6 is another example of a process 600 of performing collateral circulation assessment using a deep learning model. FIG. 6 is an example of an ensemble model. Therefore, FIG. 6 is an example of using individual models (three models) for each image type.

In the case of the ensemble model, each model should be trained to predict the collateral circulation state using different types of training data (arterial phase images, capillary phase images, and venous phase images).

The analysis device generates the arterial phase images through the above-described process (511), and inputs the generated arterial phase images to a first model to calculate a predicted probability value of 1 (512). In this case, when the arterial phase images are composed of a plurality of slices, the analysis device may input the individual slices sequentially and determine the calculated summed average value as the predicted probability value of 1.

The analysis device generates the capillary phase images through the above-described process (521), and inputs the generated capillary phase images to a second model to calculate a predicted probability value of 2 (522). In this case, when the capillary phase images are composed of a plurality of slices, the analysis device may input the individual slices sequentially and determine the calculated summed average value as the predicted probability value of 2.

The analysis device generates the venous phase images through the above-described process (531), and inputs the generated venous phase images to a third model to calculate a predicted probability value of 3 (532). In this case, when the venous phase images are composed of a plurality of slices, the analysis device may input the individual slices sequentially and determine the calculated summed average value as the predicted probability value of 3.

The analysis device may determine the final probability value by averaging the generated predicted probability values (540). The analysis device may compare the final probability value and a cutoff value to determine the final analysis result (poor or good).

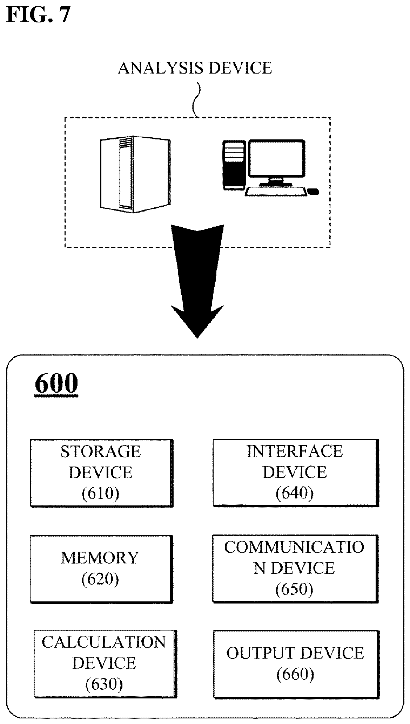

FIG. 7 is an example of an analysis device 600 for performing collateral circulation assessment using dynamic perfusion MRI. The analysis device 600 corresponds to the above-described analysis device (130 and 140 in FIG. 1). The analysis device 600 may be physically implemented in various forms. For example, the analysis device 600 may have the form of a computer device such as a PC, a server of a network, a data processing-only chipset, or the like.

The analysis device 600 includes a storage device 610, a calculation device 630, and may include a memory 620, an interface device 640, a communication device 650, and an output device 660.

The storage device 610 stores the deep learning model that predicts the above-described collateral circulation state.

The storage device 610 may store the dynamic perfusion MRI images of the subject.

The storage device 610 may store a program or code that processes the dynamic perfusion MRI images of the subject to generate the arterial phase images, the capillary phase images, and the venous phase images.

The storage device 610 may store the arterial phase images, the capillary phase images, and the venous phase images generated from the dynamic perfusion MRI images of the subject. Each image may be composed of a plurality of slices.

The storage device 610 may store analysis results (image, classification results).

The memory 620 may store data, information, and the like generated in the process of, by the analysis device 600, processing the dynamic perfusion MRI images, performing the collateral circulation assessment using the deep learning model, etc.

The interface device 640 is a device that receives predetermined commands and data from the outside. The interface device 640 may receive the dynamic perfusion MRI images to be analyzed from a physically connected input device or an external storage device. The interface device 640 may transmit analysis results (image, text, etc.) to an external object.

The communication device 650 is a configuration for receiving and transmitting predetermined information through a wired or wireless network. The communication device 650 may receive the dynamic perfusion MRI images to be analyzed from the external object. Alternatively, the communication device 650 may transmit the analysis results (image, text, etc.) to an external object such as a user terminal.

The interface device 640 and the communication device 650 are configured to transmit and receive certain data from a user or other physical object, and therefore, may also be collectively referred to as input/output devices. When limited to the function of receiving the dynamic perfusion MRI images, the interface device 640 and the communication device 650 may be referred to as input devices.

The output device 660 is a device that outputs predetermined information. The output device 660 may output an interface necessary for a data processing process, an analysis result, and the like.

The calculation device 630 may receive the dynamic perfusion MRI images using the commands or program codes stored in the storage device 610 and evaluate the collateral circulation of the subject.

The calculation device 630 from the dynamic perfusion MRI images using the above-described process and corresponding program

The calculation device 630 may generate the arterial phase images, the capillary phase images, and the venous phase images.

The calculation device 630 inputs slices of the arterial phase images, the capillary phase images, and the venous phase images to the deep learning model using the above-described process and corresponding program to calculate the predicted probability value of the collateral circulation assessment for the corresponding image or slice.

The calculation device 630 may determine the final predicted probability value using the predicted probability values for the plurality of slices, as in the process described in FIG. 5 or 6.

In addition, the calculation device 630 may compare the final predicted probability value and the cutoff value to determine the collateral circulation assessment result (good or poor state) of the subject.

The calculation device 630 may be a device such as a processor, an application processor, or a chip in which a program that processes data and processes a predetermined operation is embedded.

In addition, the method of processing dynamic perfusion MRI images and the method of collateral circulation assessment described above may be implemented as a program (or application) including an executable algorithm that may be executed on a computer. The program may be stored and provided in a non-transitory computer readable medium.

The non-transitory computer-readable medium is not a medium that stores data therein for a while, such as a register, a cache, a memory, or the like, but a medium that semi-permanently stores data therein and is readable by a device. Specifically, various applications or programs described above may be provided by being stored in non-transitory readable media such as a compact disc (CD), a digital video disc (DVD), a hard disk, a Blu-ray disc, a Universal Serial Bus (USB), a memory card, a read-only memory (ROM), a programmable read only memory (PROM), an erasable PROM (EPROM), an electrically erasable PROM (EEPROM), or a flash memory.

The transitory readable media are of various RAMs such as a static RAM (SRAM), a dynamic RAM (DRAM), a synchronous DRAM (SDRAM), a double data rate SDRAM (DDR SDRAM), an enhanced SDRAM (ESDRAM), a synclink DRAM (SLDRAM), and a direct rambus RAM (DRRAM).

The scope of protection is limited to the appended claims.

## Claims

1. A method of collateral circulation assessment of a subject using dynamic perfusion magnetic resonance imaging (MRI) images, the method comprising:
receiving, by an analysis device (600), dynamic perfusion MRI images of a brain of a subject (210) from an electronic medical record (120);
extracting, by the analysis device (600), arterial phase images, capillary phase images, and venous phase images from the dynamic perfusion MRI images (220);
inputting, by the analysis device (600), the arterial phase images into a first pre-trained deep learning model to calculate a first probability value for a collateral circulation state (512);
inputting, by the analysis device (600), the capillary phase images into a second pre-trained deep learning model to calculate a second probability value for the collateral circulation state (522);
inputting, by the analysis device (600), the venous phase images into a third pre-trained deep learning model to calculate a third probability value for the collateral circulation state (532); and
performing, by the analysis device (600), a final collateral circulation assessment for the subject using the first probability value , the second probability value and the third probability value (540); wherein
the extracting of the arterial phase images, the capillary phase images, and the venous phase images includes:
converting, by the analysis device (600), MRI signal intensity of the dynamic perfusion MRI images into a contrast agent concentration for each voxel (330);
calculating, by the analysis device (600), an R₂* time intensity curve (340) as an average value of contrast agent concentration for voxels of at least some of all slices in the dynamic perfusion MRI images having the contrast agent concentration;
setting, by the analysis device (600), a baseline section, an arterial phase section, a capillary phase section, and a venous phase section in the R2* time intensity curve (350); and
subtracting, by the analysis device (600), a baseline image from each of the slices located in the arterial phase section, the capillary phase section, and the venous phase section to generate the arterial phase images, the capillary phase images, and the venous phase images (360). -

2. The method of claim 1, wherein
the analysis device (600) is configured to set a section with a value within half of a maximum value in the R₂* time intensity curve as the arterial phase section at an end of the baseline section,
set a section with a value exceeding half of the maximum value as the capillary phase section at an end of the arterial phase section, and
set a section continuing from an end of the capillary phase section as the venous phase section.

3. The method of claim 1, wherein the analysis device (600) is configured to perform the collateral circulation assessment by comparing an average value of the first probability value, the second probability value and the third probability value with a threshold value.

4. An analysis device for collateral circulation assessment of a subject using dynamic perfusion magnetic resonance imaging (MRI) images, the analysis device comprising:
an input device configured to receive dynamic perfusion MRI images of a brain of a subject (210);
a storage device (610) configured to store deep learning models trained to calculate a collateral circulation state probability value for an input MRI slice; and
a calculation device (630) configured to
extract arterial phase images, capillary phase images, and venous phase images from the dynamic perfusion MRI images (220);
input the arterial phase images into a first deep learning model to calculate a first probability value for a collateral circulation state (512);
input the capillary phase images into a second deep learning model to calculate a second probability value for the collateral circulation state (522);
input the venous phase images into a third deep learning model to calculate a third probability value for the collateral circulation state (532); and
perform a final collateral circulation assessment for the subject using the first probability value , the second probability value and the third probability values (540), wherein the calculation device (630) is configured to convert MRI signal intensity of the dynamic perfusion MRI images into a contrast agent concentration for each voxel (330);
calculate an R₂* time intensity curve (340) as an average value of the contrast agent concentration for voxels of at least some of all slices in the dynamic perfusion MRI images having the contrast agent concentration,
set a baseline section, an arterial phase section, a capillary phase section, and a venous phase section in the R₂* time intensity curve (350), and
subtract a baseline image from each of the slices located in the arterial phase section, the capillary phase section, and the venous phase section to generate the arterial phase images, the capillary phase images, and the venous phase images (360).

5. The analysis device of claim 4, wherein the calculation device (630) is configured to
set a section having a value within half of a maximum value in the R₂* time intensity curve at an end of the baseline section as the arterial phase section,
set a section having a value within half of a maximum value at an end of the arterial phase section as the capillary phase section, and
set a section continuing from an end of the capillary phase section as the venous phase section.

6. The analysis device of claim 4, wherein the calculation device (630) is configured to perform the collateral circulation assessment by comparing an average value of the first probability value , the second probability value and the third probability value with a threshold value.

## Patentansprüche

1. Verfahren zur Bewertung der Kollateralzirkulation eines Probanden unter Verwendung dynamischer Perfusions-MRT-Bilder, wobei das Verfahren umfasst:
Empfangen, durch eine Auswertevorrichtung (600), dynamischer Perfusions-MRT-Bilder eines Gehirns eines Probanden (210) aus einer elektronischen Patientenakte (120);
Extrahieren, durch die Auswertevorrichtung (600), von Bildern der arteriellen Phase, Bildern der kapillären Phase und Bildern der venösen Phase aus den dynamischen Perfusions-MRT-Bildern (220);
Eingeben, durch die Auswertevorrichtung (600), der Bilder der arteriellen Phase in ein erstes vortrainiertes Deep-Learning-Modell, um einen ersten Wahrscheinlichkeitswert für einen Kollateralstatus zu berechnen (512);
Eingeben, durch die Auswertevorrichtung (600), der Bilder der kapillären Phase in ein zweites vortrainiertes Deep-Learning-Modell, um einen zweiten Wahrscheinlichkeitswert für den Kollateralstatus zu berechnen (522);
Eingeben, durch die Auswertevorrichtung (600), der Bilder der venösen Phase in ein drittes vortrainiertes Deep-Learning-Modell, um einen dritten Wahrscheinlichkeitswert für den Kollateralstatus zu berechnen (532); und
Durchführen, durch die Auswertevorrichtung (600), einer abschließenden Bewertung der Kollateralzirkulation des Probanden unter Verwendung des ersten Wahrscheinlichkeitswerts, des zweiten Wahrscheinlichkeitswerts und des dritten Wahrscheinlichkeitswerts (540);
wobei das Extrahieren der Bilder der arteriellen Phase, der Bilder der kapillären Phase und der Bilder der venösen Phase umfasst:
Umwandeln, durch die Auswertevorrichtung (600), einer MRT-Signalintensität der dynamischen Perfusions-MRT-Bilder in eine Kontrastmittelkonzentration für jedes Voxel (330);
Berechnen, durch die Auswertevorrichtung (600), eines R2*-Intensitätsverlaufs (340) als Mittelwert der Kontrastmittelkonzentration für Voxel zumindest eines Teils sämtlicher Schichten der dynamischen Perfusions-MRT-Bilder, die die Kontrastmittelkonzentration aufweisen;
Festlegen, durch die Auswertevorrichtung (600), eines Referenzabschnitts, eines Abschnitts der arteriellen Phase, eines Abschnitts der kapillären Phase und eines Abschnitts der venösen Phase in dem R2*-Intensitätsverlauf (350); und
Subtrahieren, durch die Auswertevorrichtung (600), eines Referenzbildes von jeder der Schichten, die sich in dem Abschnitt der arteriellen Phase, dem Abschnitt der kapillären Phase und dem Abschnitt der venösen Phase befinden, um die Bilder der arteriellen Phase, die Bilder der kapillären Phase und die Bilder der venösen Phase zu erzeugen (360).

2. Verfahren nach Anspruch 1, wobei die Auswertevorrichtung (600) dazu eingerichtet ist,
einen Abschnitt, der in dem R2*-Intensitätsverlauf einen Wert innerhalb der Hälfte eines Maximalwerts aufweist, am Ende des Referenzabschnitts als den Abschnitt der arteriellen Phase festzulegen,
einen Abschnitt, der einen die Hälfte des Maximalwerts überschreitenden Wert aufweist, am Ende des Abschnitts der arteriellen Phase als den Abschnitt der kapillären Phase festzulegen, und
einen Abschnitt, der sich an ein Ende des Abschnitts der kapillären Phase anschließt, als den Abschnitt der venösen Phase festzulegen.

3. Verfahren nach Anspruch 1, wobei die Auswertevorrichtung (600) dazu eingerichtet ist, die Bewertung der Kollateralzirkulation durch Vergleichen eines Mittelwerts des ersten Wahrscheinlichkeitswerts, des zweiten Wahrscheinlichkeitswerts und des dritten Wahrscheinlichkeitswerts mit einem Schwellenwert durchzuführen.

4. Auswertevorrichtung zur Bewertung der Kollateralzirkulation eines Probanden unter Verwendung dynamischer Perfusions-MRT-Bilder, wobei die Auswertevorrichtung umfasst:
eine Eingabeeinrichtung, die dazu eingerichtet ist, dynamische Perfusions-MRT-Bilder eines Gehirns eines Probanden (210) zu empfangen;
eine Speichereinrichtung (610), die dazu eingerichtet ist, Deep-Learning-Modelle zu speichern, die darauf trainiert sind, einen Wahrscheinlichkeitswert für einen Kollateralstatus für eine eingegebene MRT-Schicht zu berechnen; und
eine Berechnungseinrichtung (630), die dazu eingerichtet ist,
Bilder der arteriellen Phase, Bilder der kapillären Phase und Bilder der venösen Phase aus den dynamischen Perfusions-MRT-Bildern zu extrahieren (220),
die Bilder der arteriellen Phase in ein erstes Deep-Learning-Modell einzugeben, um einen ersten Wahrscheinlichkeitswert für einen Kollateralstatus zu berechnen (512),
die Bilder der kapillären Phase in ein zweites Deep-Learning-Modell einzugeben, um einen zweiten Wahrscheinlichkeitswert für den Kollateralstatus zu berechnen (522),
die Bilder der venösen Phase in ein drittes Deep-Learning-Modell einzugeben, um einen dritten Wahrscheinlichkeitswert für den Kollateralstatus zu berechnen (532), und
eine abschließende Bewertung der Kollateralzirkulation des Probanden unter Verwendung des ersten Wahrscheinlichkeitswerts, des zweiten Wahrscheinlichkeitswerts und des dritten Wahrscheinlichkeitswerts durchzuführen (540),
wobei die Berechnungseinrichtung (630) dazu eingerichtet ist,
eine MRT-Signalintensität der dynamischen Perfusions-MRT-Bilder in eine Kontrastmittelkonzentration für jedes Voxel umzuwandeln (330),
einen R2*-Intensitätsverlauf (340) als Mittelwert der Kontrastmittelkonzentration für Voxel zumindest eines Teils sämtlicher Schichten der dynamischen Perfusions-MRT-Bilder, die die Kontrastmittelkonzentration aufweisen, zu berechnen,
einen Referenzabschnitt, einen Abschnitt der arteriellen Phase, einen Abschnitt der kapillären Phase und einen Abschnitt der venösen Phase in dem R2*-Intensitätsverlauf festzulegen (350), und
ein Referenzbild von jeder der Schichten, die sich in dem Abschnitt der arteriellen Phase, dem Abschnitt der kapillären Phase und dem Abschnitt der venösen Phase befinden, zu subtrahieren, um die Bilder der arteriellen Phase, die Bilder der kapillären Phase und die Bilder der venösen Phase zu erzeugen (360).

5. Auswertevorrichtung nach Anspruch 4, wobei die Berechnungseinrichtung (630) dazu eingerichtet ist,
einen Abschnitt, der in dem R2*-Intensitätsverlauf einen Wert innerhalb der Hälfte eines Maximalwerts aufweist, am Ende des Referenzabschnitts als den Abschnitt der arteriellen Phase festzulegen,
einen Abschnitt, der einen Wert innerhalb der Hälfte des Maximalwerts aufweist, am Ende des Abschnitts der arteriellen Phase als den Abschnitt der kapillären Phase festzulegen, und
einen Abschnitt, der sich an ein Ende des Abschnitts der kapillären Phase anschließt, als den Abschnitt der venösen Phase festzulegen.

6. Auswertevorrichtung nach Anspruch 4, wobei die Berechnungseinrichtung (630) dazu eingerichtet ist, die Bewertung der Kollateralzirkulation durch Vergleichen eines Mittelwerts des ersten Wahrscheinlichkeitswerts, des zweiten Wahrscheinlichkeitswerts und des dritten Wahrscheinlichkeitswerts mit einem Schwellenwert durchzuführen.

## Revendications

1. Procédé pour évaluation de circulation collatérale d'un sujet en utilisant des images d'imagerie par résonance magnétique (IRM) de perfusion dynamique, le procédé comprenant:
recevoir, au moyen d'un dispositif d'analyse (600), des images IRM de perfusion dynamique du cerveau d'un sujet (210) provenant d'un dossier médical électronique (120);
extraire, par le dispositif d'analyse (600), des images de la phase artérielle, des images de la phase capillaire et des images de la phase veineuse à partir des images d'IRM de perfusion dynamique (220);
introduire, par le dispositif d'analyse (600), les images de la phase artérielle dans un premier modèle d'apprentissage profond pré-entraîné afin de calculer une première valeur de probabilité pour un état de la circulation collatérale (512);
introduire, par le dispositif d'analyse (600), les images de la phase capillaire dans un second modèle d'apprentissage profond pré-entraîné afin de calculer une seconde valeur de probabilité pour l'état de la circulation collatérale (522);
introduire, par le dispositif d'analyse (600), les images de la phase veineuse dans un troisième modèle d'apprentissage profond pré-entraîné afin de calculer une troisième valeur de probabilité de l'état de la circulation collatérale (532);
réaliser, par le dispositif d'analyse (600), une évaluation finale de la circulation collatérale du sujet en utilisant la première valeur de probabilité, la deuxième valeur de probabilité et la troisième valeur de probabilité (540); dans lequel
l'extraction des images de la phase artérielle, des images de la phase capillaire et des images de la phase veineuse comprend:
convertir, par le dispositif d'analyse (600), l'intensité du signal IRM des images d'IRM de perfusion dynamique en une concentration d'agent de contraste pour chaque voxel (330);
calculer, par le dispositif d'analyse (600), une courbe d'intensité temporelle R₂^{*} (340) en tant que valeur moyenne de la concentration d'agent de contraste pour les voxels d'au moins une partie de l'ensemble des coupes des images d'IRM de perfusion dynamique ayant la concentration d'agent de contraste;
définir, par le dispositif d'analyse (600), une section de base, une section de la phase artérielle, une section de la phase capillaire et une section de la phase veineuse dans la courbe d'intensité temporelle R2* (350); et
soustraire, par le dispositif d'analyse (600), une image de base de chacune des coupes situées dans la section de la phase artérielle, la section de la phase capillaire et la section de la phase veineuse, afin de générer les images de la phase artérielle, les images de la phase capillaire et les images de la phase veineuse (360).

2. Procédé selon la revendication 1, dans lequel le dispositif d'analyse (600) est configuré pour
définir une section présentant une valeur correspondant à la moitié d'une valeur maximale dans la courbe d'intensité temporelle R₂* comme section de la phase artérielle à une extrémité de la section de base,
définir une section présentant une valeur dépassant la moitié de la valeur maximale comme section de la phase capillaire à une extrémité de la section de la phase artérielle, et
définir une section se poursuivant à partir d'une extrémité de la section de la phase capillaire comme section de la phase veineuse.

3. Procédé selon la revendication 1, dans lequel le dispositif d'analyse (600) est configuré pour
effectuer l'évaluation de la circulation collatérale en comparant une valeur moyenne de la première valeur de probabilité, de la deuxième valeur de probabilité et de la troisième valeur de probabilité avec une valeur seuil.

4. Dispositif d'analyse destiné à l'évaluation de la circulation collatérale d'un sujet en utilisant d'images d'imagerie par résonance magnétique (IRM) de perfusion dynamique, le dispositif d'analyse comprenant :
un dispositif d'entrée configuré pour recevoir des images d'IRM de perfusion dynamique du cerveau d'un sujet (210);
un dispositif de stockage (610) configuré pour mémoriser des modèles d'apprentissage profond entraînés à calculer une valeur de probabilité de l'état de la circulation collatérale pour une coupe IRM d'entrée; et
un dispositif de calcul (630) configuré pour extraire, à partir des images d'IRM de perfusion dynamique (220), des images de la phase artérielle, des images de la phase capillaire et des images de la phase veineuse;
introduire les images de la phase artérielle dans un premier modèle d'apprentissage profond afin de calculer une première valeur de probabilité d'un état de la circulation collatérale (512);
introduire les images de la phase capillaire dans un second modèle d'apprentissage profond afin de calculer une seconde valeur de probabilité pour l'état de la circulation collatérale (522);
introduire les images de la phase veineuse dans un troisième modèle d'apprentissage profond afin de calculer une troisième valeur de probabilité de l'état de la circulation collatéral (532); et
effectuer une évaluation finale de la circulation collatérale pour le sujet en utilisant la première valeur de probabilité, la deuxième valeur de probabilité et la troisième valeur de probabilité (540), dans lequel
le dispositif de calcul (630) est configuré pour convertir l'intensité du signal IRM des images d'IRM de perfusion dynamique en une concentration d'agent de contraste pour chaque voxel (330);
calculer une courbe d'intensité temporelle R₂* (340) en tant que valeur moyenne de la concentration de l'agent de contraste pour les voxels d'au moins une partie de l'ensemble des coupes des images d'IRM de perfusion dynamique ayant la concentration d'agent de contraste,
définir une section de base, une section de la phase artérielle, une section de la phase capillaire et une section de la phase veineuse dans la courbe d'intensité temporelle R₂* (350), et
soustraire une image de base de chacune des coupes situées dans la section de la phase artérielle, la section de la phase capillaire et la section de la phase veineuse, afin de générer les images de la phase artérielle, les images de la phase capillaire et les images de la phase veineuse (360).

5. Dispositif d'analyse selon la revendication 4, dans lequel le dispositif de calcul (630) est configuré pour
définir, à une extrémité de la section de base, une section ayant une valeur correspondant à la moitié d'une valeur maximale dans la courbe d'intensité temporelle R₂* comme section de la phase artérielle,
définir une section ayant une valeur correspondant à la moitié de la valeur maximale à une extrémité de la section de la phase artérielle comme section de la phase capillaire, et
définir une section se poursuivant à partir d'une extrémité de la section de la phase capillaire comme section de la phase veineuse.

6. Dispositif d'analyse de la revendication 4, dans lequel le dispositif de calcul (630) est configuré pour
effectuer l'évaluation de la circulation collatérale en comparant une valeur moyenne de la première valeur de probabilité, de la deuxième valeur de probabilité et de la troisième valeur de probabilité avec une valeur seuil.
